# DEMANDE DE BREVET EUROPEEN

(11) **EP 3 329 841 A1**
(43) Date de publication de la demande: **06.06.2018**
(21) Numéro de dépôt: 17182220.8
(22) Date de dépôt: 19.07.2017
(51) Int. Cl.: A61B 5/00, A61B 5/107, A61B 5/11, G01B 7/02

(54) **DISPOSITIF DE DETECTION DE POSITION ANORMALE ET D'ALERTE ET PROCEDE ASSOCIE**

(30) Priorité: 19.07.2016 FR 1656887
(71) Demandeur: Intellidom, 19100 Brive La Gaillarde (FR)
(72) Inventeur: Collier, Paul-Louis, 19520 Cublac (FR)
(74) Mandataire: Cabinet Chaillot

(57) **Abrégé**

La présente invention a pour objet un dispositif de détection de position anormale et d'alerte (1) comprenant un boîtier (2) et des moyens de fixation (3) du boîtier (2), le boîtier (2) comprenant une batterie, un dispositif de calcul et des moyens de détection de distance, des moyens d'alerte et des moyens d'interface (32), le boîtier (2) étant monté rotatif par rapport aux moyens de fixation (3), le boîtier (2) comprenant en outre un lest, les moyens de détection de distance comprenant un premier moyen de détection de distance pour mesurer une première distance selon une première direction et un second moyen de détection de distance pour mesurer une seconde distance dans une seconde direction perpendiculaire à la première direction, les première et seconde directions définissant un plan orthogonal à l'axe de rotation de boîtier, le dispositif de calcul étant configuré pour activer les moyens d'alerte en fonction d'au moins l'une de la première et de la deuxième distances mesurées, les moyens d'interface (32) étant configurés pour activer ou acquitter les moyens d'alerte.

## Description

La présente invention porte sur un dispositif de détection de position anormale et d'alerte, en particulier un dispositif de détection de position anormale pour personnes âgées ou pour personnes handicapées, ainsi que sur un procédé associé.

Lors du maintien à domicile des personnes âgées ou des personnes handicapées, il est nécessaire de surveiller ces personnes et plus particulièrement de s'assurer qu'en cas de chute, des secours puissent intervenir le plus rapidement possible. Pour ce faire, il est courant de recourir à des dispositifs qui détectent une chute et alertent des proches ou des organismes de secours comme suite à la détection de la chute.

Il existe de nombreux dispositifs qui sont portés par la personne à surveiller et qui reposent sur la détection de paramètres actimétriques, par exemple des accéléromètres, grâce auxquels la vitesse d'accélération détectée indique une chute. De telles données peuvent également être couplées à des données d'orientation issues d'un gyroscope, comme décrit par exemple dans la demande de brevet chinois CN103027687.

Toutefois, de tels capteurs d'accélération ne détectent pas une chute lente ou progressive.

En outre, le modèle d'utilité chinois CN204274428 et la demande de brevet chinois CN104224182 divulguent l'utilisation de capteurs de hauteur pour déterminer une possible chute du porteur. De tels capteurs fonctionnent par ultrasons et mesurent une distance par rapport au sol. Lorsque la valeur mesurée est inférieure à une valeur seuil, un module de communication à distance envoie un message d'alerte pour indiquer une possible chute.

Toutefois, bien que le capteur détecte une valeur de hauteur en dehors d'une gamme prédéfinie, la corrélation entre la hauteur mesurée et la réalité d'une situation de chute n'est pas toujours fiable, dans la mesure où le porteur peut être simplement en position normale assise.

Par ailleurs, il est courant que de tels dispositifs soient paramétrés pour ne détecter qu'une position à proximité du sol. De tels dispositifs ne permettent donc pas de discriminer une position normale, à savoir debout ou assis, d'une autre position dite anormale. Ainsi, lorsque le porteur, comme suite à un malaise ou un faux mouvement par exemple, est bloqué dans une position intermédiaire entre la station debout et la position allongée, de tels dispositifs demeurent muets et donc inefficaces.

Il existe donc un besoin pour un dispositif d'alerte qui soit capable de déterminer à la fois une chute lente ou progressive et une position anormale de porteur, et ce de façon fiable.

Le demandeur se propose de résoudre ces problèmes par l'emploi d'un dispositif de détection de position anormale et d'alerte qui est configuré pour, dans un premier temps, détecter une distance par rapport au sol, et en cas de valeur anormale détectée, dans un second temps, détecter la présence d'un éventuel dossier pour confirmer ou infirmer une position assise et donc la détection d'une chute.

La présente invention a donc pour objet un dispositif de détection de position et d'alerte comprenant un boîtier et des moyens de fixation du boîtier au niveau des flancs d'un porteur humain, le boîtier comprenant une batterie alimentant d'une part un dispositif de calcul et d'autre part des moyens de détection de distance, des moyens d'alerte et des moyens d'interface connectés au dispositif de calcul, caractérisé par le fait que le boîtier est monté rotatif par rapport aux moyens de fixation selon un axe de rotation de boîtier, le boîtier comprenant en outre un lest monté dans le boîtier de telle sorte que le centre de gravité du lest est excentré par rapport à l'axe de rotation de boîtier pour que le poids du lest exerce un moment par rapport à l'axe de rotation de boîtier pour faire tourner le boîtier par rapport à l'axe de rotation de boîtier, les moyens de détection de distance comprenant un premier moyen de détection de distance pour mesurer une première distance selon une première direction et un second moyen de détection de distance pour mesurer une seconde distance dans une seconde direction perpendiculaire à la première direction, les première et seconde directions définissant un plan orthogonal à l'axe de rotation de boîtier, la première direction correspondant à la direction du poids du lest et la première distance étant mesurée dans le sens allant de l'axe de rotation de boîtier vers le centre de gravité du lest, la seconde direction de détection étant destiné à être orientée vers l'arrière du porteur P quel que soit le flanc sur lequel est fixé le dispositif, le dispositif de calcul étant configuré pour activer les moyens d'alerte en fonction d'au moins l'une de la première et de la deuxième distances mesurées, les moyens d'interface étant configurés pour activer ou acquitter les moyens d'alerte.

Selon un mode de réalisation particulier, le dispositif de détection de position et d'alerte comprend en outre des moyens de communication à distance connectés au dispositif de calcul et configurés pour transmettre des informations à un terminal à distance.

De tels moyens de communication à distance sont avantageux pour informer automatiquement, sans intervention du porteur, des tiers que le porteur se trouve dans une position anormale ou a chuté, afin de leur permettre ainsi d'intervenir rapidement.

Selon un mode de réalisation particulier, le dispositif de calcul est configuré pour activer sélectivement le second moyen de détection de distance en fonction d'une distance mesurée par le premier moyen de détection de distance.

Un tel fonctionnement en deux temps permet de confirmer ou d'infirmer la valeur anormale mesurée par le premier moyen de détection de distance.

Selon un mode de réalisation particulier, le dispositif de calcul est configuré pour activer les moyens d'alerte avec le porteur en fonction d'une distance mesurée par le second moyen de détection de distance, puis pour activer les moyens de communication à distance en l'absence d'interaction du porteur avec les moyens d'interface.

L'utilisation de moyens d'alerte et de moyens d'interface autorise le porteur qui, bien que les première seconde distances soient des valeurs anormales, ne se trouverait pas une position anormale, à annuler le signal d'alerte pour éviter de prévenir les tiers inutilement.

Selon un mode de réalisation particulier, les premier et second moyens de détection de distance sont l'un parmi des émetteurs-récepteurs à ultrasons et des émetteurs-récepteurs du type à ondes UWB.

Selon un mode de réalisation particulier, dans le plan défini par les première et deuxième directions, le boîtier a une forme choisie parmi une forme ogivale, ayant une partie pointe et une partie rectiligne opposée à la partie pointe par rapport au centre du boîtier, l'axe de rotation de boîtier étant situé au niveau de la pointe, et une forme de coeur, l'axe de rotation de boîtier étant situé au niveau de la pointe.

De telles formes sont particulièrement avantageuse d'un point de vue ergonomique car elles permettent une prise en main commode du dispositif.

Selon un mode de réalisation particulier, les moyens d'interface sont constitués par un ou plusieurs boutons disposés à la surface du boîtier, au moins l'un des boutons étant de préférence un microrupteur situé à l'intersection de la surface du boîtier et de la première direction et servant à acquitter les moyens d'alerte.

Un tel positionnement permet au porteur de trouver rapidement le microrupteur dès qu'il a en main le boîtier, sans même avoir besoin de regarder le boîtier.

Selon un mode de réalisation particulier, les moyens d'alerte sont constitués par au moins l'un parmi un ou plusieurs assourdisseurs, un ou plusieurs haut-parleurs, et une ou plusieurs diodes électroluminescentes.

Selon un mode de réalisation particulier, les moyens de communication à distance sont choisis parmi des puces de communication à protocole GSM ®, SigFox ®, Bluetooth ®, ZigBee ®, Z-Wave ®.

Selon un mode de réalisation particulier, le dispositif de calcul est au moins l'un parmi un processeur de signaux numériques, un microprocesseur, un microcontrôleur, une matrice prédiffusée programmable (FPGA) ou un composant à application spécifique (ASIC).

Selon un mode de réalisation particulier, le dispositif de détection de position et d'alerte comprend en outre des moyens de détection actimétrique, de préférence un accéléromètre.

Selon un mode de réalisation particulier, le dispositif de détection de position et d'alerte comprend en outre des moyens de détection de posture, de préférence un gyroscope six axes.

De tels moyens de détection actimétrique et de posture sont particulièrement avantageux pour fournir des informations complémentaires quant à une chute ou une position anormale du porteur.

Selon un mode de réalisation particulier, le dispositif de détection de position et d'alerte comprend en outre au moins un capteur choisi parmi des capteurs de gaz et des capteurs de température.

Selon un mode de réalisation particulier, la surface extérieure du boîtier porte un revêtement antidérapant.

Ainsi, le boîtier est aisément et sûrement pris en main par le porteur, en particulier lorsque ce dernier est affaibli.

Selon un mode de réalisation particulier, le moyen de fixation est constitué par une pince, configurée pour se pincer sur une ceinture ou la taille d'un pantalon.

La présente invention a également pour objet un procédé de détection de position et d'alerte utilisant un dispositif de détection de position et d'alerte tel que décrit ci-dessus, caractérisé par le fait qu'il comprend les étapes consistant à :
a) configurer dans le dispositif de calcul une première distance de référence et une seconde distance de référence,
b) mesurer une première distance dans la première direction, à savoir vers le sol, par l'intermédiaire du premier moyen de détection de distance,
c) comparer, par l'intermédiaire du dispositif de calcul, la première distance mesurée à la première distance de référence,
d) si la première distance mesurée est inférieure à la première distance de référence, activer le second moyen de détection de distance, sinon revenir à l'étape b),
e) mesurer une seconde distance dans la seconde direction, à savoir vers l'arrière du porteur, par l'intermédiaire du second moyen de détection de distance,
f) comparer, par l'intermédiaire du dispositif de calcul, la seconde distance mesurée à la seconde distance de référence,
g) si la seconde distance mesurée est supérieure à la seconde distance de référence, activer les moyens d'alerte, sinon revenir à l'étape b).

Le procédé peut comprendre en outre l'étape consistant à, en l'absence d'acquittement des moyens d'alerte par interaction du porteur avec les moyens d'interface, émettre par l'intermédiaire des moyens de communication à distance un message d'alerte, sinon revenir à l'étape a).

Pour mieux illustrer l'objet de la présente invention, on va en décrire ci-après, à titre illustratif et non limitatif, un mode de réalisation particulier, avec référence aux dessins annexés.

Sur ces dessins :
- la Figure 1 est une vue en perspective du dispositif de détection de position anormale de porteur et d'alerte ;
- la Figure 2 est une vue en coupe transversale du dispositif de la Figure 1 ;
- la Figure 3 est une vue en coupe longitudinale du dispositif de la Figure 1 selon l'axe AA ;
- la Figure 4 est une vue de face du dispositif de la Figure 1 en configuration désassemblée ;
- la Figure 5 est une vue de côté d'un porteur équipé du dispositif de la Figure 1 en position debout ;
- la Figure 6 est une vue de côté du porteur équipé du dispositif de la Figure 1 en position assise sur une chaise ;
- la Figure 7 est une vue de côté du porteur équipé du dispositif de la Figure 1 en position à genoux ;
- la Figure 8 est une vue de face du dispositif de la Figure 1 portant une représentation schématique des modules ;
- la Figure 9 est un ordinogramme de fonctionnement du dispositif de la Figure 1.

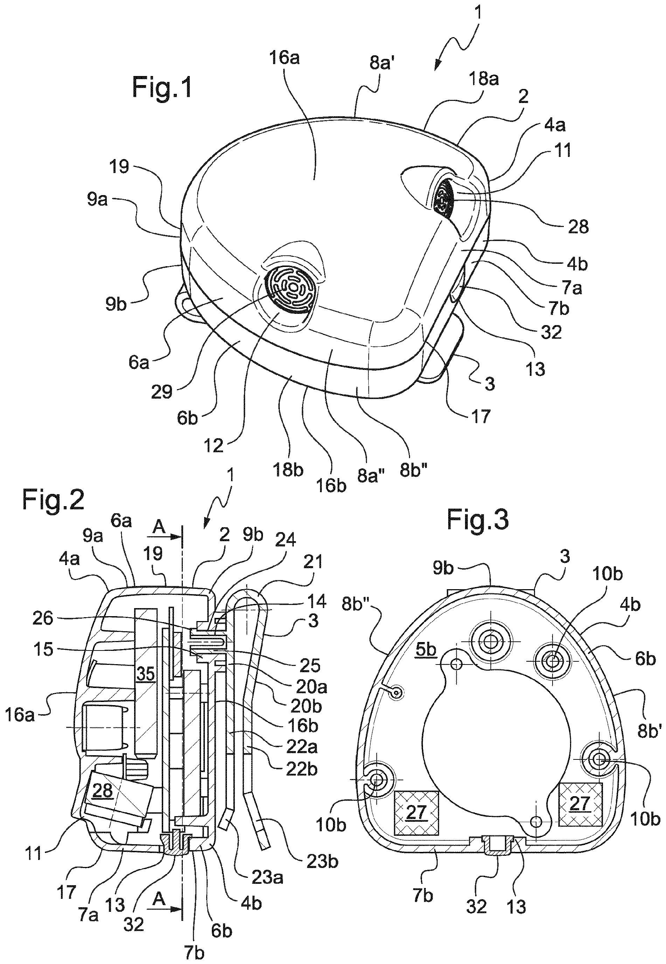

Si l'on se réfère à la Figure 1, on peut voir que l'on y a représenté un dispositif de détection de position anormal de porteur et d'alerte 1 selon la présente invention.

Le dispositif de détection de position anormale de porteur et d'alerte 1 comporte un boîtier 2 et des moyens de fixation 3 du boîtier 2 à un porteur.

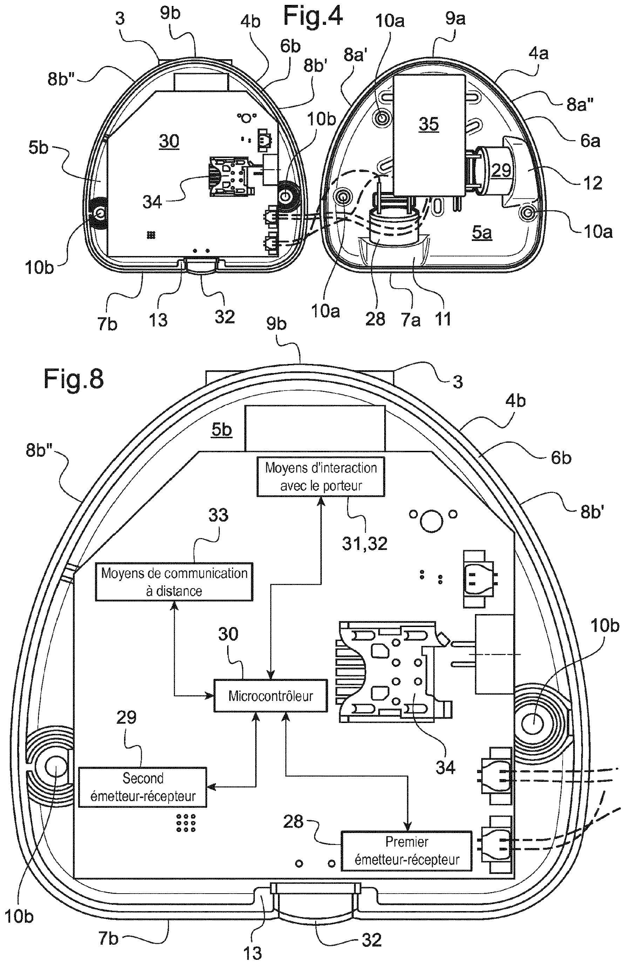

Le boîtier 2 comporte deux demi-coques 4a, 4b creuses telles qu'on peut les voir sur la Figure 4, destinées à être assemblées pour constituer le boîtier 2. Les deux demi-coques 4a, 4b sont constituées par une demi-coque avant 4a et une demi-coque arrière 4b. On entend par arrière le côté orienté vers le porteur et par avant le côté orienté à l'opposé du porteur, en utilisation, lorsque le dispositif de détection de position anormale de porteur et d'alerte 1 est porté par le porteur.

Les demi-coques 4a, 4b sont de forme concave, comprenant un fond 5a, 5b et un bord relevé 6a, 6b perpendiculaire par rapport au fond 5a, 5b. Sur la Figure 1, on peut voir que l'arête formée par le fond 5a, 5b et le bord relevé 6a, 6b est chanfreinée. On pourrait envisager une arête droite ou arrondie. De préférence, tel qu'on peut le voir sur la vue en coupe transversale de la Figure 2, le fond 5b de la demi-coque arrière 4b est plat.

Egalement, vues de face sur la Figure 4, les deux demi-coques 4a, 4b présentent une forme ogivale. Ainsi, les bords relevés 6a, 6b présentent une portion rectiligne 7a, 7b, deux portions arquées 8a', 8a", 8b', 8b" partant depuis les extrémités de la portion rectiligne 7a, 7b et se rejoignant en une portion de pointe 9a, 9b opposée à la portion rectiligne 7a, 7b par rapport au centre de la demi-coques 4a, 4b.

Il convient de noter que la forme ogivale des demi-coques 4a, 4b n'est pas limitative et que toute autre forme pourrait convenir, par exemple une forme de coeur présentant ainsi un point de rebroussement opposé à la portion de pointe par rapport au centre de la demi-coque 4a, 4b.

Les deux demi-coques 4a, 4b comprennent des moyens d'accouplement 10a, 10b configurés pour solidariser les deux demi-coques 4a, 4b. Sur la Figure 4, on peut voir que les moyens d'accouplement 10a, 10b sont constitués par des parties mâles 10a s'étendant depuis la face intérieure du fond 5a de la demi-coque avant 4a et des parties femelles 10b s'étendant depuis la face intérieure du fond 5b de la demi-coque arrière 4b, et positionnées de façon à correspondre aux parties mâles 10a. De tels moyens d'accouplement 10a, 10b sont du type bien connu dans le domaine de l'assemblage de boîtiers. Ils sont configurés pour permettre un assemblage rapide, commode et sûr du boîtier 2, ainsi que son démontage, par exemple en cas de maintenance. Tous types couramment utilisés sont envisageables.

Si l'on se réfère à nouveau à la Figure 1, on peut voir que la demi-coque avant 4a comprend une première ouverture circulaire 11 et une seconde ouverture circulaire 12. La première ouverture 11 est positionnée au niveau de la portion rectiligne 7a, et à proximité de l'intersection entre la portion rectiligne 7a et une première portion arquée 8a'. La seconde ouverture 12 est positionnée au niveau de l'autre portion arquée 8a". Le rôle des première 11 et seconde 12 ouvertures sera explicité plus loin dans la description.

Sur la Figure 1, on peut voir que la demi-coque arrière 4b comporte au centre de la portion rectiligne 8b une ouverture 13 sensiblement circulaire. Egalement, la demi-coque arrière 4b comporte une ouverture circulaire 14 pratiquée dans le fond 5b, à proximité de la portion de pointe 9b comme on peut le voir sur la Figure 2. Toujours sur la Figure 2, on peut voir que l'ouverture 14 comporte des parois 15 qui font saillie des deux côtés du fond 5b, générant ainsi un cylindre creux.

Le rôle des ouvertures 13 et 14 sera explicité plus loin dans la description.

Pour assembler le boîtier 2, les deux demi-coques 4a, 4b sont rapprochées, côté ouvert en regard, les bords relevés 6a, 6b, mis en correspondance. Ainsi, en configuration assemblée, le boîtier 2 comporte sur sa surface extérieure, et définies par les fonds 5a, 5b, une face avant 16a et une face arrière 16b. De plus, en configuration assemblée, le boîtier comprend une portion rectiligne 17 par l'intermédiaire des portions rectilignes 7a, 7b des bords relevés 6a, 6b, des portions arquées 18a, 18b par l'intermédiaire des bords arqués 8a', 8b', 8a", 8b", partant des extrémités de la portion rectiligne 17 et se rejoignant en une portion de pointe 19, définie par l'intermédiaire des portions de pointe 9a, 9b, opposées à la portion rectiligne 17 par rapport au centre du boîtier 2.

En utilisation, la portion de pointe 19 est dirigée vers la tête du porteur et la portion rectiligne 17 est orientée vers le sol.

D'autre part, la forme concave des deux demi-coques 4a, 4b définit à l'intérieur du boîtier 2 un volume de réception fermé.

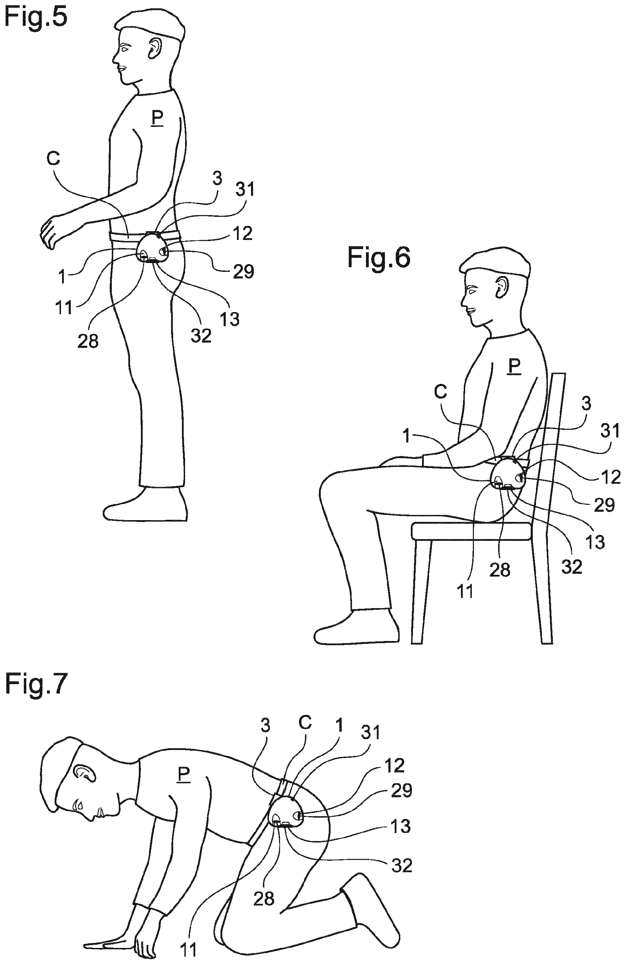

Les moyens de fixation 3 du dispositif 1 au porteur sont représentés plus particulièrement sur la Figure 2, représentant une vue en coupe transversale du dispositif 1. De tels moyens de fixation 3 sont du type à pince. Ils sont fixés au niveau de la face arrière 16b du boîtier 2. Ils comprennent une paire de bras 20a, 20b de forme sensiblement parallélépipédique, reliés au niveau d'une extrémité par une liaison de type charnière 21 pour former une pince. Les bras 20a, 20b comportent deux parties centrales 22a, 22b, parallèles entre elles et à la face arrière 16b du boîtier 2. De façon connue, la charnière 21 exerce une force de compression de façon à rapprocher les parties centrales parallèles 22a, 22b, de façon à pouvoir maintenir le dispositif 1 en pinçant les habits du porteur, en particulier en clipsant les moyens de fixation 3 sur une ceinture C tel que représenté sur les Figures 5-7. On pourrait envisager de remplacer la liaison de type charnière 21 par une liaison pivot ou comportant un ressort de compression sans s'écarter du cadre de la présente invention. En outre les faces en regard des bras 20a, 20b peuvent comprendre un revêtement antidérapant ou des nervures de façon à accroître l'adhérence et donc la fixation.

De préférence, tel que représenté sur la Figure 2, les bras 20a, 20b comprennent des parties terminales 23a, 23b s'écartant l'une de l'autre depuis la partie centrale 20a, 20b vers leur extrémité libre. Une telle conception permet d'une part de faire passer aisément la zone de fixation, une ceinture par exemple, du dispositif 1 entre les bras 20a, 20b, et d'autre part, facilite l'écartement des bras 20a, 20b afin de détacher le dispositif 1 du porteur.

Pour un fonctionnement optimal du dispositif 1, ce dernier est attaché par l'intermédiaire des moyens de fixation 3 au niveau des flancs du porteur P. On entend par flancs toute partie latérale du corps. De préférence, le dispositif 1 est fixé au niveau de la hanche du porteur P, comme on peut le voir sur les Figures 5-7.

Le dispositif 1 comprend également des moyens de rotation 24 configurés pour permettre la rotation du boîtier 2 par rapport aux moyens de fixation 3. On peut voir sur la Figure 2 que les moyens de rotation 24 comprennent un axe de rotation cylindrique 25. L'axe de rotation 25 est positionné dans l'ouverture 15 de la demi-coque arrière 4b et son diamètre extérieur est légèrement inférieur au diamètre de l'ouverture 15 de façon à pouvoir tourner librement dans l'ouverture cylindrique 15. L'axe de rotation 25 comprend, côté volume intérieur du boîtier 2, des retours 26 en appui contre les extrémités des parois 14 de l'ouverture 15 de façon à empêcher la sortie de l'axe de rotation 25 hors de l'ouverture 15. De façon avantageuse, l'axe de rotation 25 est réalisé en une matière semi-rigide de façon à faciliter l'insertion de l'axe de rotation 25 dans l'ouverture 15.

L'axe de rotation 25 est également fixé sur les moyens de fixations 3 du dispositif 1 au porteur. On peut voir sur la Figure 2 que l'axe de rotation 25 est fixé à proximité de la charnière 21 des moyens de fixation 3, sur le bras 20a positionné côté face arrière 16b du boîtier 2.

L'axe de rotation 25 est orienté perpendiculairement aux parties centrales 22a, 22b des bras 20a, 20b, et à la face arrière 16b du boîtier 2, de sorte que le boîtier 2 et les moyens de fixation 3 tournent dans deux plans parallèles, orthogonaux à l'axe de rotation 25.

Sur la Figure 2, on peut voir que l'axe de rotation 25 est d'un seul tenant avec les moyens de fixation 3. On pourrait envisager que l'axe de rotation 25 soit maintenu par un autre moyen, par collage ou clipsage par exemple.

Sur la Figure 3, on peut voir que l'on a représenté une vue de face de la demi-coque arrière 4b. Sur le côté intérieur du fond 5b de la demi-coque 4b est fixé un lest 27. Le lest 27 est composé de deux éléments de lest 27 qui sont positionnés à proximité de la portion droite 7b. Il convient de noter que l'emploi d'un unique élément de lest 27 pourrait convenir. Le centre de gravité du lest 27 est excentré par rapport à l'axe de rotation 25 pour que le poids du lest 27 exerce un moment par rapport à l'axe de rotation 25 pour faire tourner le boîtier 2 par rapport à l'axe de rotation 25, dans un plan de rotation orthogonal à l'axe de rotation 25, et ainsi amener le boîtier 2 à pivoter par rapport aux moyens de fixation. Ainsi, pour un fonctionnement optimal, le centre de gravité du lest 27 est situé éloigné de l'axe de pivotement 25. Une première direction est ainsi définie dans un plan de rotation vertical, et orientée dans le sens allant de l'axe de pivotement 25 vers le centre de gravité du lest 27, c'est-à-dire correspondant à la direction du poids du lest 27. Ainsi, en utilisation, grâce à la liaison pivotante du boîtier 2 par rapport aux moyens de fixation 3, la direction d'orientation est en permanence verticale et orientée vers le sol.

Le dispositif 1 comprend également des premier 28 et second 29 moyens de détection de distance configurés pour détecter une distance. Les premier 28 et second 29 moyens de détection de distance sont constitués par un premier émetteur-récepteur 28 et un second émetteur-récepteur 29. Sur la Figure 1, on peut voir que le premier émetteur-récepteur 28 est positionné dans la première ouverture 11 du boîtier 2 et le second émetteur-récepteur 29 est positionné dans la seconde ouverture 12 du boîtier 2.

Le premier émetteur-récepteur 28 est configuré pour émetteur et recevoir en retour un signal selon la première direction de détection contenue dans le plan de rotation du boîtier 2, correspondant à la direction du poids du lest 27. Ainsi, en utilisation, comme on peut le voir sur les Figures 5-7, le premier émetteur-récepteur 28 est en permanence dirigé vers le sol. Le premier émetteur-récepteur 28 est ainsi configuré pour détecter une distance du dispositif 1 par rapport au sol.

Le second émetteur-récepteur 29 est configuré pour émetteur et recevoir en retour un signal selon une seconde direction de détection contenue dans le plan de rotation du boîtier 2, perpendiculaire à la première direction de détection du premier émetteur-récepteur 28, et orientée vers l'arrière du porteur P. Ainsi, en utilisation, comme on peut le voir sur les Figures 5-7, lorsque le porteur P fixe le dispositif 1 au niveau de ses flancs, la seconde direction de détection est dirigée en permanence vers l'arrière du porteur P. Le second émetteur-récepteur 29 est ainsi configuré pour déterminer une distance du dispositif 1, et donc du porteur P par rapport à un objet se situant à l'arrière du porteur P, comme par exemple un dossier de chaise tel que représenté sur la Figure 6.

Les premier 28 et second 29 émetteurs-récepteurs sont de préférence du type à ultrasons ou du type à ondes UWB. On pourrait envisager, sans s'écarter du cadre de la présente invention, que les premier 28 et second 29 émetteurs-récepteurs soient de tous types connus comme par exemple à infra-rouge.

Il convient de noter que, de façon avantageuse, le boîtier 2 présente une forme ogivale dans le plan de rotation du boîtier 2, contenant les première et seconde directions de détection.

Le dispositif 1 comprend également, logé à l'intérieur du volume de réception du boîtier 2, un dispositif de calcul 30. Le dispositif de calcul 30 est configuré pour commander sélectivement les premier 28 et second 29 émetteurs-récepteurs et comparer les distances détectées par les premier 28 et second 29 émetteurs-récepteurs à des valeurs ou des plages prescrites. Le dispositif de calcul 30 sera avantageusement au moins l'un parmi un processeur de signaux numériques, un microprocesseur, un microcontrôleur, une matrice prédiffusée programmable (FPGA) ou un composant à application spécifique (ASIC). Selon le mode de réalisation représenté, le dispositif de calcul 30 est un microcontrôleur 30.

Le dispositif 1 comprend également des moyens d'interaction avec le porteur 31, 32. Les moyens d'interaction avec le porteur sont configurés pour émettre une alerte et annuler l'alerte. Les moyens d'interaction 31, 32 comprennent des moyens d'alerte 31 et des moyens d'interface 32.

Les moyens d'alerte 31 peuvent être des moyens d'alerte visuelle 31 et/ou des moyens d'alerte sonore. Sur les Figures 5 à 7, les moyens d'alerte visuelle 31 représentés comprennent une diode électroluminescente. La diode 31 fait saillie hors du boîtier 2, au niveau de la portion de pointe 19, dans un orifice ménagé dans le boîtier 2. Ainsi, elle est directement visible par le porteur. Les moyens d'alerte 31 sont configurés pour émettre une alerte au porteur et sont commandés par le microcontrôleur 30.

Selon un mode de réalisation non représenté, les moyens d'alerte sont constitués par l'un parmi un ou plusieurs assourdisseurs, un ou plusieurs haut-parleurs.

Les moyens d'interface 32 sont configurés pour activer ou acquitter les moyens d'alerte 31.

Sur le dispositif 1 représenté sur les Figures 1-7, les moyens d'interface 32 comprennent un microrupteur 32. Le microrupteur 32 est positionné dans l'ouverture 13 ménagée dans la portion rectiligne 17 du boîtier 2. Pour un repérage rapide, le microrupteur 32 est positionné à l'intersection de la surface du boîtier 2 et de la première direction de détection. Sur les Figures 1-8, on peut voir que le microrupteur 32 est positionné au centre de la portion rectiligne 17 de façon à être aisément et rapidement accessible par le porteur, tout en évitant tout risque que le microrupteur 32 soit déclenché inopinément lors d'une chute. Ainsi, le microrupteur 32 est actionné uniquement par action volontaire de l'utilisateur, ce qui limite le risque qu'une alerte ne soit pas émise alors que l'utilisateur a chuté. Lorsque le boîtier 2 est de forme ogivale comme sur les Figures 5 à 7, la prise en main est commode et, du fait du positionnement du microrupteur 32 au centre de la portion rectiligne 17, qui en utilisation du dispositif 1 est orientée vers le sol, le porteur peut presser le microrupteur 32 d'un simple geste et annuler l'alerte rapidement. Selon un mode de réalisation non représenté, le boîtier 2 est sous forme de coeur, le microrupteur 32 étant positionné de façon avantageuse au niveau du point de rebroussement. Dans de telles configurations, les doits du porteur trouvent rapidement le microrupteur 32 sans même avoir besoin de regarder le boîtier 2. Lorsque le porteur actionne les moyens d'interface 32, le dispositif de calcul 30 désactive les moyens d'alerte 31.

De façon avantageuse, la surface extérieure du boîtier 2 comprend un revêtement antidérapant pour une prise en main commode du dispositif 1.

Le dispositif 1 comprend en outre des moyens de communication à distance 33. Les moyens de communication à distance 33 sont commandés par le microcontrôleur 30 et sont configurés pour transmettre des informations, en particulier les valeurs de distance mesurées par les moyens de détection de distance 28, 29, et émettre un signal d'alerte à un terminal à distance informant d'une chute ou d'une position anormale du porteur. Les moyens de communication à distance sont choisis parmi des puces de communication à protocole GSM ®, SigFox ®, Bluetooth ®, ZigBee ®, Z-Wave ®.

Tel qu'on peut le voir sur les Figure 4 et 8, les premier 28 et second 29 émetteurs-récepteurs, le microcontrôleur 30, les moyens d'alerte 31, les moyens d'interface 32, et les moyens de communication à distance 33 sont connectés au moyen d'une carte imprimée 34. Par ailleurs, comme on peut le voir sur les Figures 2 et 4, les premier 28 et second 29 émetteurs-récepteurs, le microcontrôleur 30, les moyens d'alerte 31, les moyens d'interface 32, et les moyens de communication à distance 33 sont alimentés par l'intermédiaire d'un batterie 35. La batterie 35 est constituée par tous types de batterie couramment rencontrés, et de préférence une batterie amovible de façon à pouvoir être remplacée. Une ou plusieurs batteries lithium-ion ou même des piles classiques peuvent être envisagées pour jouer le rôle de la batterie. Dans le cas d'une batterie rechargeable, un port de charge pourra être prévu sur le boîtier pour permettre de recharger la batterie sans avoir à ouvrir le boîtier.

Selon un mode de réalisation non représenté, le dispositif comprend des moyens de détection complémentaires tels que des moyens de détection actimétrique de type accéléromètre, des moyens de détection de posture de type gyroscope six axes ou encore des capteurs choisis parmi des capteurs de gaz et des capteurs de température.

Des tels dispositifs sont connectés au microcontrôleur 30 et sont particulièrement avantageux pour fournir des informations complémentaires quant à la position du porteur et à son environnement, à transmettre par l'intermédiaire des moyens de communication à distance 33. On pourrait envisager que les informations détectés par les moyens de détection de distance 28, 29 et les moyens de détection complémentaires soient affichées au porteur par l'intermédiaire d'un dispositif d'affichage connecté au microcontrôleur 30.

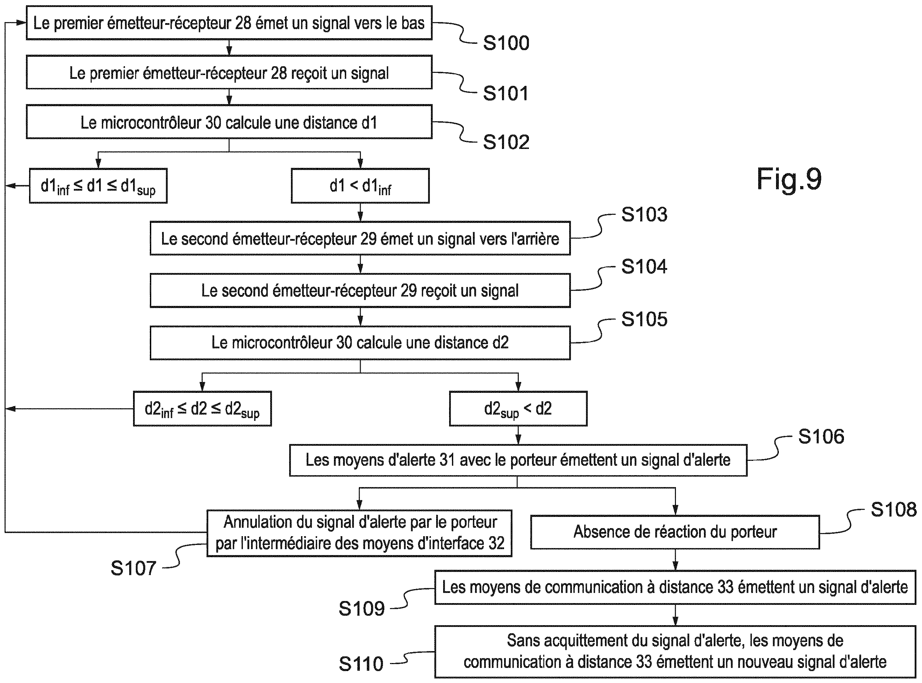

Nous allons maintenant décrire ci-après le fonctionnement du dispositif 1 à l'appui des Figures 5-7 et 9.

Comme on peut le voir sur la Figure 5, en utilisation, le dispositif de détection de position anormale de porteur et d'alerte 1, de forme ogivale, est fixé au niveau de la hanche du porteur P, sur sa ceinture C, par l'intermédiaire des moyens de fixation 3. Le dispositif 1 est orienté de telle sorte que la portion de pointe 19 soit dirigée vers la tête du porteur P en station debout, et la portion rectiligne 17 orientée vers le sol. Du fait du lest 27 porté par le boîtier 2, le premier-émetteur récepteur 28 est orienté selon la direction du poids du lest 27, à savoir vers le sol. Le microcontrôleur 30 active le premier émetteur-récepteur 28 qui émet un signal vers le bas (S100) en direction du sol, le second émetteur-récepteur 29, les moyens d'alerte 31, les moyens d'interface 32 et les moyens de communication à distance 33 étant désactivés. On peut envisager que le microcontrôleur 30 commande l'activation du premier émetteur-récepteur 28 en permanence ou à intervalles prédéfinis.

En retour, le premier émetteur-récepteur 28 reçoit un signal (S101) provenant du sol. Sur la base des signaux émis et reçus, le microcontrôleur 30 calcule une première distance d1 (S102) du dispositif 1 par rapport au sol et compare la première distance d1 à une première plage prédéfinie dans le microcontrôleur 30. Si la première distance d1 est comprise entre les bornes inférieure d1_{inf} et supérieure d1ₛᵤₚ de la première plage prédéfinie, le microcontrôleur 30 continue l'activation du premier émetteur-récepteur 28.

Si la première distance d1 est inférieure à la borne inférieure d1_{inf} de la première plage prédéfinie, à savoir que le dispositif 1, donc les hanches du porteur P, se trouve à proximité du sol comme sur les Figures 6 et 7, indiquant un chute probable, le microcontrôleur 30 active le second émetteur-récepteur 29 qui émet un signal vers l'arrière (S103) du porteur P. Lorsque le second émetteur-récepteur 29 reçoit en retour un signal (S104), le microcontrôleur 30 calcule une seconde distance d2 (S105) du dispositif 1 par rapport à un objet se trouvant à l'arrière du porteur P et compare la seconde distance d2 à une seconde plage prédéfinie dans le microcontrôleur 30. Si la seconde distance d2 est comprise entre les bornes inférieure d2_{inf} et supérieure d2ₛᵤₚ de la seconde plage prédéfinie, à savoir que le dispositif 1, donc les hanches du porteur P, est à proximité d'un objet, à savoir un dossier de chaise et donc que le porteur P est dans une position normale assise comme représenté sur la Figure 6, le microcontrôleur 30 annule l'activation du second émetteur-récepteur 29 et continue l'activation du premier émetteur-récepteur 28.

Si la seconde distance d2 est supérieure à la borne supérieure d2ₛᵤₚ de la seconde plage prédéfinie, à savoir qu'il n'y a pas d'objet à proximité du dos du porteur P, donc pas de dossier de chaise sur laquelle serait assis le porteur P, le microcontrôleur 30 active les moyens d'alerte 31 qui émettent une alerte au porteur P (S106). Par exemple, la diode électroluminescente 31 clignote ou le haut-parleur émet une sonnerie. Si le porteur P n'a pas chuté, ni ne se trouve dans une position anormale, par exemple il est momentanément accroupi, il annule le signal d'alerte par l'intermédiaire des moyens d'interface 32 (S107), par exemple en pressant le microrupteur 32. Pour ce faire, il lui suffit de saisir le boîtier 2 dans le creux de sa main, ce qui est rendu aisé par la forme ogivale du boîtier 2, et de presser le microrupteur 32 ainsi placé au niveau de ses doigts. Dans ce cas, le microcontrôleur 30 désactive les moyens d'alerte 31 et continue l'activation du premier émetteur-récepteur 28.

En l'absence d'annulation d'alerte par le porteur P (S108), par exemple après l'écoulement d'un intervalle temporel prédéfini dans le microcontrôleur 30, le microcontrôleur 30 active les moyens de communication à distance 33 qui émettent un signal d'alerte (S109), voire également transmettent les informations détectées par les moyens de détection de distance 28, 29 et d'éventuels moyens de détection complémentaires, vers un terminal à distance. En l'absence d'acquittement du signal d'alerte par le terminal/le dispositif distant/la personne auquel il est envoyé au bout d'un nombre d'envois d'alerte prédéterminé, les moyens de communication à distance 33 envoient le signal d'alerte sur un autre terminal/dispositif distant ou à une autre personne (S110).

On pourrait envisager que le dispositif 1 fonctionne selon un mode dans lequel le microcontrôleur 30 active les moyens d'alerte 31 en fonction de la première distance d1 détectée par le premier émetteur-récepteur 28, puis les moyens de communication à distance 33 en l'absence d'interaction du porteur P avec les moyens d'interface 32.

Par ailleurs, on pourrait envisager un mode de réalisation dans lequel le dispositif 1 comprend un interrupteur actionné par le porteur pour activer/désactiver le dispositif 1.

Avantageusement, avec le mode de fonctionnement décrit ci-dessus, la batterie est économisée car le deuxième moyen de détection de distance n'est activé, et donc consomme l'énergie de la batterie, uniquement dans les cas où le premier moyen de détection de distance indique une possibilité de position anormale du porteur P.

La présente invention a également pour objet un procédé de détection de position anormale et de d'alerte utilisant un dispositif de détection de position anormale et d'alerte tel que décrit ci-dessus, caractérisé par le fait qu'il comprend les étapes consistant à :
a) configurer dans le dispositif de calcul une première distance de référence et une seconde distance de référence,
b) mesurer une première distance dans la première direction par l'intermédiaire du premier moyen de détection de distance,
c) comparer, par l'intermédiaire du dispositif de calcul, la première distance mesurée à la première distance de référence,
d) si la première distance mesurée est inférieure à la première distance de référence, activer le second moyen de détection de distance, sinon revenir à l'étape b),
e) mesurer une seconde distance dans la seconde direction par l'intermédiaire du second moyen de détection de distance,
f) comparer, par l'intermédiaire du dispositif de calcul, la seconde distance mesurée à la seconde distance de référence,
g) si la seconde distance mesurée est supérieure à la seconde distance de référence, activer les moyens d'alerte, sinon revenir à l'étape b).

## Revendications

1. Dispositif de détection de position et d'alerte (1) comprenant un boîtier (2) et des moyens de fixation (3) du boîtier (2) au niveau des flancs d'un porteur humain (P), le boîtier (2) comprenant une batterie (35) alimentant d'une part un dispositif de calcul (30) et d'autre part des moyens de détection de distance (28, 29), des moyens d'alerte (31) et des moyens d'interface (32) connectés au dispositif de calcul (30), **caractérisé par le fait que** le boîtier (2) est monté rotatif par rapport aux moyens de fixation (3) selon un axe de rotation de boîtier (25), le boîtier (2) comprenant en outre un lest (27) monté dans le boîtier (2) de telle sorte que le centre de gravité du lest (27) est excentré par rapport à l'axe de rotation de boîtier (25) pour que le poids du lest (27) exerce un moment par rapport à l'axe de rotation de boîtier (25) pour faire tourner le boîtier (2) par rapport à l'axe de rotation de boîtier (25), les moyens de détection de distance (28, 29) comprenant un premier moyen de détection de distance (28) pour mesurer une première distance selon une première direction et un second moyen de détection de distance (29) pour mesurer une seconde distance dans une seconde direction perpendiculaire à la première direction, les première et seconde directions définissant un plan orthogonal à l'axe de rotation de boîtier (25), la première direction correspondant à la direction du poids du lest (27) et la première distance étant mesurée dans le sens allant de l'axe de rotation de boîtier (25) vers le centre de gravité du lest (27), la seconde direction de détection étant destinée à être orientée vers l'arrière du porteur P quel que soit le flanc sur lequel est fixé le dispositif (1), le dispositif de calcul (30) étant configuré pour activer les moyens d'alerte (31) en fonction d'au moins l'une de la première et de la deuxième distances mesurées, les moyens d'interface (32) étant configurés pour activer ou acquitter les moyens d'alerte (31).

2. Dispositif de détection de position et d'alerte (1) selon la revendication 1, **caractérisé par le fait qu'**il comprend en outre des moyens de communication à distance (33) connectés au dispositif de calcul (30) et configurés pour transmettre des informations à un terminal à distance.

3. Dispositif de détection de position et d'alerte (1) selon la revendication 1 ou la revendication 2, **caractérisé par le fait que** le dispositif de calcul (30) est configuré pour activer sélectivement le second moyen de détection de distance (29) en fonction d'une distance mesurée par le premier moyen de détection de distance (28).

4. Dispositif de détection de position et d'alerte (1) selon l'une des revendications 2 et 3, **caractérisé par le fait que** le dispositif de calcul (30) est configuré pour activer les moyens d'alerte (31) avec le porteur en fonction d'une distance mesurée par le second moyen de détection de distance (29), puis pour activer les moyens de communication à distance (33) en l'absence d'interaction du porteur avec les moyens d'interface (32).

5. Dispositif de détection de position et d'alerte (1) selon l'une des revendications 1 à 4, **caractérisé par le fait que** les premier (28) et second (29) moyens de détection de distance sont l'un parmi des émetteurs-récepteurs à ultrasons et des émetteurs-récepteurs du type à ondes UWB.

6. Dispositif de détection de position et d'alerte (1) selon l'une des revendications 1 à 5, **caractérisé par le fait que** dans le plan défini par les première et deuxième directions, le boîtier (2) a une forme choisie parmi une forme ogivale, ayant une partie pointe (19) et une partie rectiligne (17) opposée à la partie pointe (19) par rapport au centre du boîtier (2), l'axe de rotation de boîtier (25) étant situé au niveau de la pointe (19), et une forme de coeur, l'axe de rotation de boîtier (25) étant situé au niveau de la pointe (19).

7. Dispositif de détection de position et d'alerte (1) selon l'une des revendications 1 à 6, **caractérisé par le fait que** les moyens d'interface (31) sont constitués par un ou plusieurs boutons (31) disposés à la surface du boîtier (2), au moins l'un des boutons (31) étant de préférence un microrupteur (31) situé à l'intersection de la surface du boîtier (2) et de la première direction et servant à acquitter les moyens d'alerte (31).

8. Dispositif de détection de position et d'alerte (1) selon l'une des revendications 1 à 7, **caractérisé par le fait que** les moyens d'alerte (31) sont constitués par au moins l'un parmi un ou plusieurs assourdisseurs, un ou plusieurs haut-parleurs, et une ou plusieurs diodes électroluminescentes (31).

9. Dispositif de détection de position et d'alerte (1) selon l'une des revendications 1 à 8, **caractérisé par le fait que** les moyens de communication à distance (33) sont choisis parmi des puces de communication à protocole GSM ®, SigFox ®, Bluetooth ®, ZigBee ®, Z-Wave ®.

10. Dispositif de détection de position et d'alerte (1) selon l'une des revendications 1 à 8, **caractérisé par le fait que** le dispositif de calcul (30) est au moins l'un parmi un processeur de signaux numériques, un microprocesseur, un microcontrôleur, une matrice prédiffusée programmable (FPGA) ou un composant à application spécifique (ASIC).

11. Dispositif de détection de position et d'alerte (1) selon l'une des revendications 1 à 10, **caractérisé par le fait qu'**il comprend en outre des moyens de détection actimétrique, de préférence un accéléromètre.

12. Dispositif de détection de position et d'alerte (1) selon l'une des revendications 1 à 11, **caractérisé par le fait qu'**il comprend en outre des moyens de détection de posture, de préférence un gyroscope six axes.

13. Dispositif de détection de position et d'alerte (1) selon l'une des revendications 1 à 12, **caractérisé par le fait qu'**il comprend en outre au moins un capteur choisi parmi des capteurs de gaz et des capteurs de température.

14. Dispositif de détection de position et d'alerte (1) selon l'une des revendications 1 à 13, **caractérisé par le fait que** la surface extérieure du boîtier (2) porte un revêtement antidérapant.

15. Dispositif de détection de position et d'alerte (1) selon l'une des revendications 1 à 14, **caractérisé par le fait que** les moyens de fixation (3) sont constitués par une pince (3), configurée pour se pincer sur une ceinture (C) ou la taille d'un pantalon.

16. Procédé de détection de position et de d'alerte utilisant un dispositif de détection de position et d'alerte (1) selon l'une des revendications 1 à 15, **caractérisé par le fait qu'**il comprend les étapes consistant à :
a) configurer dans le dispositif de calcul (30) une première distance de référence et une seconde distance de référence,
b) mesurer une première distance dans la première direction, à savoir vers le sol, par l'intermédiaire du premier moyen de détection de distance (28),
c) comparer, par l'intermédiaire du dispositif de calcul (30, la première distance mesurée à la première distance de référence,
d) si la première distance mesurée est inférieure à la première distance de référence, activer le second moyen de détection de distance (29), sinon revenir à l'étape b),
e) mesurer une seconde distance dans la seconde direction, savoir vers l'arrière du porteur, par l'intermédiaire du second moyen de détection de distance (29),
f) comparer, par l'intermédiaire du dispositif de calcul (30), la seconde distance mesurée à la seconde distance de référence,
g) si la seconde distance mesurée est supérieure à la seconde distance de référence, activer les moyens d'alerte (31), sinon revenir à l'étape b).

17. Procédé de détection de position et d'alerte selon la revendication 16 utilisant un dispositif de détection de position et d'alerte (1) selon l'une des revendications 2 à 15, **caractérisé par le fait qu'**il comprend l'étape consistant à, en l'absence d'acquittement des moyens d'alerte (31) par interaction du porteur avec les moyens d'interface (32), émettre par l'intermédiaire des moyens de communication à distance (33) un message d'alerte, sinon revenir à l'étape a).
